# EUROPEAN PATENT SPECIFICATION

(11) **EP 3 958 897 B1**
(45) Date of publication and mention of the grant of the patent: **06.05.2026**
(21) Application number: 20724301.5
(22) Date of filing: 15.04.2020
(51) Int. Cl.: C07K 16/24, A61P 1/00

(54) **MIRIKIZUMAB FOR USE IN A METHOD OF TREATING CROHN'S DISEASE**
MIRIKIZUMAB ZUR VERWENDUNG IN EINEM VERFAHREN ZUR BEHANDLUNG VON MORBUS CROHN
MIRIKIZUMAB DESTINÉ À ÊTRE UTILISÉ DANS UN PROCÉDÉ DE TRAITEMENT DE LA MALADIE DE CROHN

(30) Priority: 22.04.2019 US 201962836910 P
(43) Date of publication of application: 02.03.2022
(73) Proprietor: Eli Lilly and Company, Indianapolis, IN 46285 (US)
(72) Inventor: FRIEDRICH, Stuart William, Indianapolis, Indiana 46206-6288 (US); POLLACK, Paul Frederick, Indianapolis, Indiana 46206-6288 (US); TUTTLE, Jay Lawrence, Indianapolis, Indiana 46206-6288 (US)
(74) Representative: Eli Lilly and Company Limited
(86) International application number: PCT/US2020/028273
(87) International publication number: WO 2020/219314

(56) References cited:
- WO-A1-2017/048901
- CHRISTOPHER MA ET AL: "Investigational drugs in phase I and phase II clinical trials targeting interleukin 23 (IL23) for the treatment of Crohn's disease", EXPERT OPINION ON INVESTIGATIONAL DRUGS, vol. 27, no. 8, 3 August 2018 (2018-08-03), UK, pages 649 - 660, XP055711425, ISSN: 1354-3784, DOI: 10.1080/13543784.2018.1506764

## Description

This invention generally relates to mirikizumab, an antibody that binds to the p19 subunit of human IL-23 for use in the treatment of Crohn's Disease (CD).

CD is a chronic disease of unknown etiology with environmental, genetic, and immunologic influences. Transmural inflammation affecting any part of the gastrointestinal tract from the mouth to the anus, usually appearing as discontinuous lesions, are normal characteristics for CD (Baumgart D C and Sandborn WJ, Lancet, Vol. 369, pages 1641-57, 2007). Symptoms include chronic diarrhoea (often bloody and containing pus or mucus), abdominal pain, weight loss, fever, fatigue, anaemia, rectal bleeding, and a feeling of fullness in the abdomen. Symptoms depend on the severity of the disease and location of the disease, with the majority of patients experiencing an abscess, fistula, stricture or an obstruction requiring surgical intervention. Relapsing-remitting symptoms, meaning that many patients have intermittent disease flares that are interspersed with periods of remission, is very common in CD (Lichtenstein G R et al., American Journal of Gastroenterology, Vol. 113, pages 481-517, 2018). Treatment goals in clinical practice are control of symptoms and healing of the intestinal mucosa.

Treatment of autoimmune/inflammatory diseases with IL-23 targeted therapy is being pursued. The first such biologic to demonstrate clinical benefit in autoimmune disease was ustekinumab, which is a Food and Drug Administration (FDA)-approved monoclonal antibody for the treatment of psoriasis, psoriatic arthritis and CD. Ustekinumab binds the common p40 subunit of IL-12 and IL-23; therefore, it targets both cytokines, rather than IL-23 specifically. Blockade of the IL-12 pathway may prevent Th1 cell-induced interferon blockade of Th17 cell development, thus potentially limiting the clinical activity of p40 targeting antibodies. Experimental studies suggest that blocking the IL-23/Th17/IL-17 immune axis alone is sufficient to treat autoimmune inflammation (Monteleone G et al., Mediators of Inflammation, E-publication, 27 May 2009). Agents specifically targeting the IL-23 p19 subunit have demonstrated clinical activity in psoriasis (Sofen H et al., J Allergy Clin Immunol. Vol. 133, No. 4, pages 1032-1040, 2014; Kopp T et al., Nature, Vol. 521, No. 7551, pages 222-226, 2015; Krueger J G et al., J Allergy Clin Immunol., Vol. 136, No. 1, pages 116-124 e7, 2015). IL-23 p19-specific antibodies have also demonstrated clinical activity in CD (Sands B E et al., Gastroenterology, Vol. 148, No. 4, Supplement 1, S163-S164, Abstract 830, 2015; Feagan B G et al., Gastroenterology, Vol. 150, No. 4, Supplement 1, S 1266, Abstract 812a, 2016).

Treatment regimens for CD with anti-IL-23p19 antibodies are disclosed in WO 2014/143540 Al, WO 2017/048901 Al and Ma, Christopher, et al. Expert Opinion on Investigational Drugs 27.8 (2018): 649-660.

There remains a need for treatment options for CD that lead to favourable outcomes for patients, for example, in terms of efficacy, safety and/or tolerability of the treatment. In particular, there remains a need for treatment options in the form of dosage regimen of mirikizumab that provides optimal efficacy in the treatment of CD.

Any reference to a method of treatment practiced on the human or animal body is to be interpreted as substances or compositions for use in such treatment.

The present invention provides mirikizumab for use in the treatment of Crohn's Disease (CD) said treatment comprising:
a) administering three induction doses of mirikizumab to the patient by intravenous injection, wherein each induction dose comprises about 900 mg of mirikizumab and wherein the three induction doses of mirikizumab are administered at about 4 week intervals; and
b) administering maintenance dose(s) of mirikizumab to the patient by subcutaneous injection at about 4 week or about 8 week intervals, wherein the first maintenance dose is administered about 4 weeks or about 8 weeks after the last induction dose is administered and wherein each maintenance dose comprises 200 mg or 300 mg of mirikizumab,
wherein the CD is moderate to severe CD.

In some embodiments of the invention the patient is conventional-failed.

In some embodiments of the invention the patient is biologic-experienced.

In some embodiments of the invention the patient is biologic-failed.

In some embodiments of the invention three induction doses of mirikizumab are administered at about 4 week intervals and the first maintenance dose is administered about 4 weeks after the last induction dose is administered.

Accordingly, in a first aspect of the present disclosure, there is a provided a method for treating CD comprising administering mirikizumab to a patient, said method comprising:
a) administering at least one induction dose of mirikizumab to the patient, wherein the induction dose comprises about 200 mg to about 1200 mg of mirikizumab; and
b) administering at least one maintenance dose of mirikizumab to the patient after the last induction dose is administered, wherein the maintenance dose comprises about 100 mg to about 600 mg of mirikizumab.

In a still further preferred embodiment of the method of the present disclosure, the method comprises:
a) administering three induction doses of mirikizumab to the patient by intravenous injection, wherein each induction dose comprises about 900 mg of mirikizumab; and
b) administering maintenance dose(s) of mirikizumab to the patient by subcutaneous injection at about 4 week or about 8 week intervals, wherein the first maintenance dose is administered about 4 weeks or about 8 weeks after the last induction dose is administered and wherein each maintenance dose comprises 200 mg or 300 mg of mirikizumab,
wherein the CD is moderate to severe CD.

In a further aspect of the present disclosure there is provided mirikizumab for use in the treatment of CD, said treatment comprising:
a) administering at least one induction dose of mirikizumab to the patient, wherein the induction dose comprises about 200 mg to about 1200 mg of mirikizumab; and
b) administering at least one maintenance dose of mirikizumab to the patient after the last induction dose is administered, wherein the maintenance dose comprises about 100 mg to about 600 mg of mirikizumab.

In an embodiment of the present invention, the CD is moderate to severe CD.

In a further embodiment of the present invention, the patient is conventional-failed.

In an alternative embodiment of the present invention, the patient is biologic-experienced.

In a further alternative embodiment of the present invention, the patient is biologic-failed.

In a still further embodiment of the present disclosure, the at least one induction dose comprises about 200 mg, about 300 mg, about 400 mg, about 500 mg, about 600 mg, about 700 mg, about 800 mg, about 900 mg, about 1000 mg, about 1100 mg or about 1200 mg of mirikizumab.

In a preferred embodiment of the present disclosure, the at least one induction dose comprises about 900 mg of mirikizumab.

In a still further embodiment of the present disclosure, one, two, three or four induction doses are administered to the patient.

In a further preferred embodiment of the present invention, three induction doses are administered to the patient at about 4-week intervals.

In an alternative further preferred embodiment of the present disclosure, the at least one induction dose is administered by intravenous infusion.

In a still embodiment of the present disclosure, if the patient has not achieved endoscopic response about 4 to about 12 weeks after the last induction dose is administered, at least one extended induction dose(s) of mirikizumab is administered to the patient, wherein the at least one maintenance dose(s) of mirikizumab is administered to the patient if the patient has achieved endoscopic response about 4 to about 12 weeks after the last extended induction dose is administered, and wherein endoscopic response is defined as a 50% reduction from baseline in SES-CD Score.

In a still further preferred embodiment of the present disclosure, the at least one extended induction dose(s) are administered to the patient if the patient has not achieved endoscopic response about 4 weeks after the last induction dose is administered.

In a still further embodiment of the present disclosure, multiple extended induction doses are administered at about 4 week intervals.

In a still further preferred embodiment of the present disclosure, three extended induction doses are administered at about 4 week intervals.

In a still further embodiment of the present disclosure, the extended induction dose(s) comprise about 200 mg, about 300 mg, about 400 mg, about 500 mg, about 600 mg, about 700 mg, about 800 mg, about 900 mg, about 1000 mg, about 1100 mg or about 1200 mg of mirikizumab.

Preferably, the extended induction dose(s) comprise about 200 mg, about 600 mg, about 900 mg or about 1000 mg of mirikizumab.

Further preferably, the extended induction dose(s) comprise(s) about 900 mg of mirikizumab.

In a still further preferred embodiment of the present disclosure, the one, two or three extended induction dose(s) are administered by intravenous infusion.

In a still further embodiment of the present disclosure, the at least one maintenance dose comprises about 100 mg, about 150 mg, about 200 mg, about 250 mg, about 300 mg, about 350 mg, about 400 mg, about 500 mg or about 600 mg of mirikizumab.

In a still further preferred embodiment of the present invention, the at least one maintenance dose comprises about 300 mg of mirikizumab.

In an alternative preferred embodiment of the present invention, the at least one maintenance dose comprises about 200 mg of mirikizumab

In a still further embodiment of the present disclosure, the at least one maintenance dose is administered 2-16 weeks after the last induction dose is administered.

In a still further embodiment of the present disclosure, the at least one maintenance dose is administered about 2 weeks, about 3 weeks, about 4 weeks, about 5 weeks, about 6 weeks, about 7 weeks, about 8 weeks, about 12 weeks or about 16 weeks after the last induction dose is administered.

In a still further preferred embodiment of the present invention, the at least one maintenance dose is administered about 4 weeks after the last induction dose is administered.

In an alternative further preferred embodiment of the present invention, the at least one maintenance dose is administered about 8 weeks after the last induction dose is administered.

In a still further embodiment of the present disclosure, multiple maintenance doses are administered to a patient and wherein the first maintenance dose is administered 2 to 16 weeks after the last induction dose is administered.

In a still further embodiment of the present disclosure, the first maintenance dose is administered about 2 weeks, about 3 weeks, about 4 weeks, about 5 weeks, about 6 weeks, about 7 weeks, about 8 weeks, about 12 weeks or about 16 weeks after the last induction dose is administered.

In a still further preferred embodiment of the present invention, the first maintenance dose is administered about 4 weeks after the last induction dose is administered.

In an alternative further preferred embodiment of the present invention, the first maintenance dose is administered about 8 weeks after the last induction dose is administered.

In a still further embodiment of the present disclosure, one or more further maintenance dose(s) are administered at about 4, about 8 or about 12 week interval(s) after administration of the first maintenance dose.

In a still further preferred embodiment of the present invention, one or more further maintenance dose(s) are administered at about 4 week interval(s) after administration of the first maintenance dose.

In an alternative further preferred embodiment of the present invention, one or more further maintenance dose(s) are administered at about 8 week interval(s) after administration of the first maintenance dose.

In a still further preferred embodiment of the present invention, the maintenance dose(s) are administered by subcutaneous injection.

In a still further preferred embodiment of the present disclosure, the treatment comprises:
a) administering three induction doses of mirikizumab to the patient by intravenous injection, wherein each induction dose comprises about 900 mg of mirikizumab; and
b) administering maintenance dose(s) of mirikizumab to the patient by subcutaneous injection at about 4 week or about 8 week intervals, wherein the first maintenance dose is administered about 4 weeks or about 8 weeks after the last induction dose is administered and wherein each maintenance dose comprises 200 mg or 300 mg of mirikizumab,
wherein the CD is moderate to severe CD.

In a still further aspect of the present disclosure there is provided use of mirikizumab in the manufacture of a medicament for use in the treatment of CD, said treatment comprising:
a) administering at least one induction dose of mirikizumab to the patient, wherein the induction dose comprises about 200 mg to about 1200 mg of mirikizumab; and
b) administering at least one maintenance dose of mirikizumab to the patient after the last induction dose is administered, wherein the maintenance dose comprises about 100 mg to about 600 mg of mirikizumab.

In an embodiment of the present invention, the CD is moderate to severe CD.

In a still further preferred embodiment of the present disclosure, the treatment comprises:
a) administering three induction doses of mirikizumab to the patient by intravenous injection, wherein each induction dose comprises about 900 mg of mirikizumab; and
b) administering maintenance dose(s) of mirikizumab to the patient by subcutaneous injection at about 4 week or about 8 week intervals, wherein the first maintenance dose is administered about 4 weeks or about 8 weeks after the last induction dose is administered and wherein each maintenance dose comprises 200 mg or 300 mg of mirikizumab,
wherein the CD is moderate to severe CD.

### BRIEF DESCRIPTION OF THE FIGURES

Figure 1 illustrates the average serum concentrations of mirikizumab during the induction period in the study described in Example 1. Average concentration estimated based on population PK analyses using the individual subject clearance values and the total dose received during the induction period. Subjects with low outlier concentrations are mainly the result of subjects that discontinued from the study and did not receive all the planned mirikizumab administrations.
Figure 2 illustrates the average serum concentrations of mirikizumab during the maintenance period in the study described in Example 1. Average concentration estimated based on population PK analyses using the individual subject clearance values and the dose received during the maintenance period.
**Figure 3** depicts population pharmacokinetic model-estimated clearance versus body weight in the study of Example 1.
**Figure 4** depicts population pharmacokinetic model-estimated central volume of distribution versus body weight in the study of Example 1.
**Figure 5** depicts a visual predictive check of model fit of Week 12 endoscopic response in the study of Example 1.
**Figure 6** depicts a visual predictive check of model fit of Week 12 endoscopic remission in the study of Example 1.
**Figure 7** illustrates a simulation of endoscopic response and endoscopic remission rates at Week 12 for mirikizumab doses and exposures of interest for the study of Example 2.

### DETAILED DESCRIPTION

There are various measurements of CD disease activity level including, but not limited to the Simple Endoscopic Score for Crohn's Disease (SES-CD)(Daperno M et al., Gastrointest Endosc., Vol. 60, No. 4, pages 505-512, 2004) and the Crohn's Disease Activity Index (CDAI).

The SES-CD is an endoscopic scoring system for CD based on 4 endoscopic variables (presence and size of ulcers, proportion of surface covered by ulcers, proportion of surface affected by disease, and presence and severity of stenosis), which are assessed in 5 ileocolonic bowel segments (ileum; right, transverse, and left colon; and rectum). Each of the 4 endoscopic variables is scored from 0 to 3: presence and size of ulcers (none = score 0; diameter 0.1 cm to 0.5 cm = score 1; 0.5 cm to 2 cm = score 2; >2 cm = score 3); extent of ulcerated surface (none = 0; <10% = 1; 10% to 30% = 2; >30% = 3); extent of affected surface (none = 0; <50% = 1; 50% to 75% = 2; >75% = 3); and presence and type of narrowing (none = 0; single, can be passed = 1; multiple, can be passed = 2; cannot be passed = 3). The grand total is obtained as the sum of all endoscopic scores across all bowel segments. Scores range from 0 to 56, with higher scores indicating more severe disease.

The Crohn's Disease Activity Index (CDAI is an 8-item disease activity measure comprised of a composite of 3 patient-reported and 5 physician-reported/laboratory items (physical signs and a laboratory parameter [hematocrit]). Patient responses are summed over a 7-day period and all items are subsequently weighted, yielding a total score range of 0 to 600 points. See Appendix 10.8 for additional descriptions of Patient Reported Outcomes (PROs)(e.g. CDAI-SF, CDAI-AP, and CDAI-wellbeing).

PROs include the following:
- Bowel Movement Count (BMC)
- Crohn's Disease Activity Index - Stool Frequency (CDAI-SF) Note: Bristol Stool Scale is used as a reference to complete CDAI-SF.
- Crohn's Disease Activity Index - Abdominal Pain (CDAI-AP)
- Crohn's Disease Activity Index - Well-Being (CDAI-well-being)
- Abdominal Pain NRS
- Urgency NRS
- Patient Global Rating of Severity (PGRS)
- Functional Assessment of Chronic Illness Therapy-Fatigue (FACIT-Fatigue)
- Inflammatory Bowel Disease Questionnaire (IBDQ)

As used herein, the term "biologic experienced" refers to patients that have been administered a biologic for example, an anti-TNF-α antibody, for the treatment of CD, in particular, for the treatment of moderate to severe CD. Such patients may or may not have been administered a conventional medicine for the treatment of CD. Conventional medicines for the treatment of CD include aminosalisates, 6-mercaptopurine (6-MP) or azathioprine (AZA), corticosteroids, 5-aminosalicylic acid (5-ASA) and steroids.

As used herein, the term "biologic-failed" refers to patients that have been administered a biologic, for example, an anti-TNF-α antibody, for the treatment of CD, in particular, for the treatment of moderate to severe CD. Such patients may or may not have been administered a conventional medicine for the treatment of CD. Conventional medicines for the treatment of CD include aminosalisates, 6-mercaptopurine (6-MP) or azathioprine (AZA), corticosteroids, 5-aminosalicylic acid (5-ASA) and steroids. Such patients have an inadequate response to, loss of response to, or are intolerant to biologic therapy for CD (such as anti-TNF antibodies). In the context of the terms "biologic-failed", inadequate response means signs and symptoms of persistently active disease despite induction treatment at the approved induction dosing that was indicated in the product label at the time of use. In the context of the term "biologic-failed", loss of response is defined as recurrence of signs and symptoms of active disease during approved maintenance dosing following prior clinical benefit (discontinuation despite clinical benefit does not qualify as having failed or being intolerant to CD biologic therapy). In the context of the term "biologic-failed", intolerance means a history of infliximab, adalimumab, certolizumab pegol, vedolizumab, natalizumab, or other approved biologics (including but not limited to infusion-related event, demyelination, congestive heart failure, or any other drug-related AE that led to a reduction in dose or discontinuation of the medication).

As used herein, the term "biologic-naive" refers to patients that have not been administered a biologic, for example, an anti-TNF-α antibody, for the treatment of CD, in particular, for the treatment of moderate to severe CD. Such patients may or may not have been administered a conventional medicine for the treatment of CD. Conventional medicines for the treatment of CD include aminosalisates, 6-mercaptopurine (6-MP) or azathioprine (AZA), corticosteroids, 5-aminosalicylic acid (5-ASA) and steroids.

As used herein, the term "conventional-failed" refers to patients who have an inadequate response to, loss of response to, or are intolerant to at least one of the following medications: 5-aminosalicylic (ASA) compounds; corticosteroids; AZA, 6-MP, or methotrexate (MTX) or CD-specific antibiotics. Conventional-failed patients have neither failed nor demonstrated an intolerance to a biologic medication (anti-TNF antibody or anti-integrin antibody) that is indicated for the treatment of CD.

As used herein, "moderate to severe CD" is defined as a diagnosis of CD for ≥3 months, have active CD and have a SES-CD score ≥7 (centrally read) for subjects with ileal colonic or ≥4 for subjects with isolated ileal disease within 14 days before the first dose of study treatment.

As used herein, "clinical benefit" is defined as having an endoscopic response (50% reduction from baseline in SES-CD score), or a 25% reduction from baseline in SES-CD score, combined with a 40% reduction from baseline in stool frequency (SF) or abdominal pain (AP) score

As used herein, "endoscopic response" is defined as a 50% reduction from baseline in SES-CD Score.

As used herein, "endoscopic remission SES-CD ≤ 4" is defined as total SES-CD score of ≤ 4 and at least a 2- point reduction versus baseline and no subscore >1

As used herein, "endoscopic remission SES-CD 0-2" is defined as a total SES-CD score of ≤ 2.

As used herein " clinical remission by PRO" is defined as an unweighted daily average SF ≤ 2.5 or ≤ 3 (number of liquid or very soft stools [as taken from the Crohn's Disease Activity Index, CDAI], defined using the Bristol Stool Scale Category 6 or 7 [Lewis and Heaton 1997], i.e., liquid or watery stools) and the unweighted daily average AP ≤1 (AP [4-point scale: 0 = none, 1 = mild, 2 = moderate, 3 = severe]) and both SF and AP no worse than baseline

As used herein, "clinical response by PRO" is defined as at least a 30% decrease in SF and/or AP and no worse than baseline.

As used herein, "clinical remission by CDAI" is defined as a CDAI score <150.

As used herein "clinical response by CDAI" is defined as a reduction in CDAI score by ≥100 points compared to baseline and/or being in clinical remission by CDAI.

As used herein, "dose" or "dosing" refers to to the administration of a substance (for example, mirikizumab) to achieve a therapeutic objective (for example, the treatment of CD).

As used herein, "induction period" refers to a period of treatment of a patient comprising administration of mirikizumab to the patient in order to induce endoscopic response, endoscopic remission SES-CD ≤ 4, endoscopic remission SES-CD 0-2, clinical remission by PRO, clinical response by PRO, clinical remission by CDAI or clinical response by CDAI, each of these terms as defined above. There is no minimum or maximum duration of the "induction period" but it is typically about 4, about 8 or about 12 weeks in duration. The end of induction period is typically an end-of-induction assessment occurring about 4 weeks or about 8 weeks after the last induction dose has been administered.

As used herein, "induction dose" refers to a first dose of mirikizumab administered to a patient in order to induce endoscopic response, endoscopic remission SES-CD ≤ 4, endoscopic remission SES-CD 0-2, clinical remission by PRO, clinical response by PRO, clinical remission by CDAI or clinical response by CDAI, each of these terms as defined above. The "induction dose" can be a single dose or, alternatively, a set of doses. The "induction dose" is administered during the induction period.

As used herein, "extended induction period" refers to a period of treatment of a patient comprising administration of mirikizumab to the patient that is required in order to induce endoscopic response, endoscopic remission SES-CD ≤ 4, endoscopic remission SES-CD 0-2, clinical remission by PRO, clinical response by PRO, clinical remission by CDAI or clinical response by CDAI, each of these terms as defined above, because endoscopic response, endoscopic remission SES-CD ≤ 4, endoscopic remission SES-CD 0-2, clinical remission by PRO, clinical response by PRO, clinical remission by CDAI, clinical response by CDAI was not achieved during an initial induction period. The "extended induction period" may be about 4, about 8 or about 12 weeks in duration.

As used herein, "extended induction dose" refers to a further induction dose of an mirikizumab administered to a patient in order to induce endoscopic response, endoscopic remission SES-CD ≤ 4, endoscopic remission SES-CD 0-2, clinical remission by PRO, clinical response by PRO, clinical remission by CDAI, clinical response by CDAI, each of these terms as defined above, because endoscopic response, endoscopic remission SES-CD ≤ 4, endoscopic remission SES-CD 0-2, clinical remission by PRO, clinical response by PRO, clinical remission by CDAI or clinical response by CDAI was not achieved during an initial induction period. The "extended induction dose" can be a single dose or, alternatively, a set of doses. There is no minimum or maximum duration of the "extended induction period but it is typically about 4, about 8 or about 12 weeks in duration. The end of extended induction period is typically an end-of-extended induction assessment occurring about 4 or about 8 weeks after the last extended induction dose has been administered. The "extended induction dose" is administered during the extended induction period.

As used herein, "maintenance period" refers to refers to a period of treatment comprising administration of mirikizumab to a patient in order to maintain a desired therapeutic effect, the desired therapeutic effect endoscopic response, endoscopic remission SES-CD ≤ 4, endoscopic remission SES-CD 0-2, clinical remission by PRO, clinical response by PRO, clinical remission by CDAI or clinical response by CDAI, each of these terms as defined above. The "maintenance period" follows the induction period or extended induction period, and, therefore, is initiated once a desired therapeutic effect - endoscopic response, endoscopic remission SES-CD ≤ 4, endoscopic remission SES-CD 0-2, clinical remission by PRO, clinical response by PRO, clinical remission by CDAI or clinical response by CDAI - is achieved.

As used herein, "maintenance dose" refers to a subsequent dose of mirikizumab administered to a patient to maintain or continue a desired therapeutic effect, namely, endoscopic response, endoscopic remission SES-CD ≤ 4, endoscopic remission SES-CD 0-2, clinical remission by PRO, clinical response by PRO, clinical remission by CDAI or clinical response by CDAI, each of these terms as defined above. A "maintenance dose" is administered subsequent to the induction dose. A "maintenance dose" can be a single dose or, alternatively, a set of doses.

As used herein, the terms "treating," "treat," or "treatment," refer to restraining, slowing, lessening, reducing, or reversing the progression or severity of an existing symptom, disorder, condition, or disease, or ameliorating clinical symptoms and/or signs of a condition. Beneficial or desired clinical results include, but are not limited to, alleviation of symptoms, diminishment of the extent of a disease or disorder, stabilization of a disease or disorder (i.e., where the disease or disorder does not worsen), delay or slowing of the progression of a disease or disorder, amelioration or palliation of the disease or disorder, and remission (whether partial or total) of the disease or disorder, whether detectable or undetectable. Those in need of treatment include those already with the disease.

As used herein "anti-IL-23p19 antibody" refers to an antibody, or fragment thereof, that binds to the p19 subunit of human IL-23 but does not bind to the p40 subunit of human IL-23. An anti-IL-23p19 antibody thus binds to human IL-23 but does not bind to human IL-12.

Mirikizumab, CAS Registry No. 1884201-71-1, is a humanized, IgG4-kappa monoclonal antibody targeting the p19 subunit of human IL-23. The antibody and methods of making same are described in US Patent No. 9,023.358.

Mirikizumab, or pharmaceutical compositions comprising the same, may be administered by parenteral routes (e.g., subcutaneous, intravenous, intraperitoneal, intramuscular, or transdermal).

The term "intravenous infusion" refers to introduction of an agent into the vein of an animal or human patient over a period of time greater than approximately 15 minutes, generally between approximately 30 to 90 minutes.

The term "subcutaneous injection" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

Pharmaceutical compositions comprising mirikizumab for use in the methods of the present invention can be prepared by methods well known in the art (e.g., Remington: The Science and Practice a/Pharmacy, 19th edition (1995), (A. Gennaro et al., Mack Publishing Co.) and comprise an antibody as disclosed herein, and one or more pharmaceutically acceptable carriers, diluents, or excipients.

### EXAMPLES

### EXAMPLE 1: CLINICAL STUDY

### Overview

A Phase 2 study may be conducted to determine whether mirikizumab, is safe and efficacious in subjects with moderate to severe CD. Such a study may evaluate safety and determine the clinical activity defined by improvement in CD activity measures and key patient-reported outcomes (PRO) measures.

### Objectives

The primary objective of such a Phase II study would be to demonstrate that treatment with mirikizumab is superior to placebo in inducing endoscopic response at Week 12. Secondary objectives may include the following:
- evaluation of the safety and tolerability of treatment with mirikizumab;
- evaluation of the effect of mirikizumab on the proportion of subjects with endoscopic response at Week 52;
- evaluation of the efficacy of mirikizumab as superior to placebo in endoscopic remission at Week 12;
- evaluation of the effect of mirikizumab on the proportion of subjects with endoscopic remission at Week 52,
- evaluation of the effect of mirikizumab as superior to placebo in PRO remission at Week 12;
- evaluation of the effect of mirikizumab on the proportion of subjects with PRO remission at Week 52;
- evaluation of the effect of mirikizumab on health outcomes/quality of life measures at Weeks 12 and 52; and
- characterization of the PK profile of mirikizumab.

Endpoints may be defined using the SES CD score. Endoscopies may be centrally read. Rates of endoscopic healing may be determined at Weeks 12 and 52. Endpoint definitions are as follows:

| | |
|---|---|
| • Endoscopic response: | ≥50% reduction from baseline in SES-CD |
| | Total score |
| • Endoscopic remission SES-CD ≤4: | SES-CD Total Score ≤4 and at least a 2-point reduction versus baseline and no subscore >1 |
| • Endoscopic remission SES-CD 0-2: | SES-CD Total Score ≤2 |
| • Clinical remission by PRO | Unweighted daily average SF ≤3 (number of liquid or very soft stools [as taken from the Crohn's Disease Activity Index, CDAI], defined using the Bristol Stool Scale Category 6 or 7 [Lewis and Heaton 1997], i.e., liquid or watery stools) and the unweighted daily average AP ≤1 (AP [4-point scale: 0 = none, 1 = mild, 2 = moderate, 3 = severe]) and both SF and AP no worse than baseline |
| • Clinical response by PRO | At least a 30% decrease in SF and/or AP and no worse than baseline |
| • Clinical remission by CDAI | CDAI score <150 |
| • Clinical response by CDAI | A reduction in CDAI score by ≥100 points compared to baseline and/or being in clinical remission by CDAI |

### Methods

This study may be a multi-centre, randomized, parallel-arm, placebo-controlled trial in which about 191 are randomized. Subjects may be stratified to the following categories, and the exact number enrolled in either group will be dependent upon the enrolment rate of each subject population:
i) A minimum of approximately 30% of subjects were naive to biologic CD therapy (including experimental biologic CD therapy); and
ii) At least 50% of the subjects were prior biologic CD therapy-experienced (including experience with experimental biologic CD therapy).
The study comprises the following periods:

### Screening (Approximately 4 Weeks):

Subjects may be evaluated for study eligibility ≤ 28 days before the baseline visit. Subjects may be eligible for the study only if they meet all of the following criteria within the screening period, which is ≤ 28 days prior to the start of study treatment, unless specifically defined:
Type of Subject and Disease Characteristics
i) Have had a diagnosis of CD for ≥3 months before baseline
ii) Have active CD as defined as absolute SF ≥4 (loose and watery stools defined as Bristol Stool Scale Category 6 or 7) and/or AP ≥ 2 at baseline have a SES-CD score ≥7 (centrally read) for subjects with ileal-colonic or ≥4 for subjects with isolated ileal disease within 14 days before the first dose of study treatment

### Prior IBD Treatment

A) Subjects must have received prior treatment for CD (according to either "a)" or "b)" below or combination of both):
   a) history of inadequate response to, or failure to tolerate treatment with aminosalicylsates, 6-mercaptopurine (6-MP) or azathioprine (AZA), oral or IV corticosteroids or history of corticosteroid dependence (an inability to successfully taper corticosteroids without return of CD)
      OR
   b) Have received treatment with ≥1 biologic agents (such as TNF antagonists, vedolizumab, experimental biologic CD therapeutics) with or without documented history of failure to respond to or tolerate such treatment:
      - The treatment must have been discontinued according to the following timeline:
         - anti-TNF therapy at least 8 weeks before baseline
         - vedolizumab treatment at least 12 weeks before baseline
         - experimental biologic CD therapy at least 8 weeks before baseline.
B) May be receiving a therapeutic dosage of the following drugs:
   a) Oral 5-aminosalicylic (ASA) compounds: if the prescribed dose has been stable for at least 3 weeks before screening colonoscopy or stopped treatment at least 3 weeks prior to screening colonoscopy;
   b) Oral corticosteroids must be at a prednisone-equivalent dose of ≤20 mg/day, or ≤ 9 mg/day of budesonide, and have been at a stable dose for at least 3 weeks prior to the screening colonoscopy. If stopping oral corticosteroid treatment prior to baseline, they must be stopped at least 3 weeks prior to screening colonoscopy;
   c) AZA, 6-MP, or methotrexate (MTX): if the prescribed dose has been stable for at least about 4 weeks before screening endoscopy. Subjects who have discontinued therapy with AZA, 6-MP, or MTX must have stopped the medication at least about 4 weeks prior to screening endoscopy to be considered eligible for enrolment.
   c) CD-specific antibiotics: if the prescribed dose has been stable about 4 weeks prior to baseline or stopped treatment at least 3 weeks prior to screening endoscopy.

### Assignment of treatment groups

Assignment to treatment groups may be determined by a computer-generated random sequence using an interactive web-response system (IWRS). To achieve between-group comparability, subjects are stratified to these arms based upon their prior therapy (below); this stratification is controlled by IWRS.
- A minimum of approximately 30% of subjects may be naive to biologic CD therapy (including experimental biologic CD therapy).
- At least 50% of the subjects may be prior biologic CD therapy-experienced (including experience with experimental biologic CD therapy).
- For Period 2, subjects assigned to mirikizumab at baseline may be randomized to either baseline treatment assignment or 300 mg SC mirikizumab Q4W - except for all subjects in the placebo group, and subjects in the mirikizumab treatment groups who have not had any improvement in SES-CD score from baseline at Week 12 (determined by the central reader), who receive 1000 mg intravenous (IV) mirikizumab Q4W. Preferably, all subjects receive IV and SC administration of either mirikizumab or placebo during Period 2 in a double-dummy design.

### Period 1 (Weeks 0 to 12):

A 12-week induction dosing period may be designed to evaluate the efficacy and safety of mirikizumab administered intravenously (IV) at Weeks 0, 4, 8. At baseline, subjects may be randomized with a 2:1:1:2 allocation across the 4 treatment arms and stratified on the basis of previous exposure to biologic therapy for treatment of CD:

| | |
|---|---|
| Mirikizumab Dose Arm 1 | 1000 mg IV Mirikizumab Q4W |
| Mirikizumab Dose Arm 2 | 600 mg IV Mirikizumab Q4W |
| Mirikizumab Dose Arm 3 | 200 mg IV Mirikizumab Q4W |
| Placebo | Placebo administered IV Q4W |

Period 1 may be designed to establish the efficacy (endoscopic changes and key PRO) and safety of mirikizumab versus placebo in subjects with moderate to severe Crohn's disease. Subjects may continue background pharmacotherapies for CD as permitted per protocol; therefore, the selection of placebo as a comparator in this subject population is justified to effectively evaluate the safety and efficacy of mirikizumab.

### Period 2 (Weeks 12 to 52):

Period 2 (Weeks 12 to 52) allows for continued evaluation of efficacy and safety with baseline treatment regimens and exploration of SC dosing - except for all subjects in the placebo group and subjects in the mirikizumab treatment groups who have not had any improvement in SES-CD score from baseline at Week 12.

Period 2 subjects may receive both IV and subcutaneously (SC) dosing to maintain blinding from Weeks 12 through 48. Dosing occurred Q4W. Randomization was stratified based on endoscopic response (i.e., achieving a 50% reduction in SES CD score from baseline).

All subjects who receive placebo in Period 1 should receive IV mirikizumab 1000 mg and SC placebo in Period 2. Patients who receive mirikizumab and who achieved an improvement, defined as any numeric decrease, in their SES CD score from baseline at Week 12 may be randomized to either continue Period 1 IV treatment assignment with SC placebo or IV placebo with SC mirikizumab 300 mg. Subjects who receive mirikizumab and who do not achieve an improvement that is, a score equal to baseline or higher, in their SES CD score should receive IV mirikizumab 1000 mg and SC placebo.

### Period 3 (Weeks 52 to 104):

Period 3 is intended to provide extension therapy for subjects considered to be receiving clinical benefit and provides longer term evaluation of safety and durability of clinical benefit.

All subjects having clinical benefit defined as having an endoscopic response (50% reduction from baseline in SES CD score), or a 25% reduction from baseline in SES CD score, combined with a 40% reduction from baseline in SF or AP score could continue on study treatment and proceed to Period 3 and receive 300 mg SC mirikizumab Q4W open label starting at Week 52 through Week 104. Subjects not receiving clinical benefit at Week 52 should discontinue treatment and enter the 16 week Follow Up period.

Alternatively, patients could proceed to Period 3 if judged to have clinical benefit per the judgment of the investigator.

### Follow-Up:

At Week 104, subjects stop treatment and ARE followed for safety for an additional 16 weeks.

### Statistical Analysis

The primary endpoint is Week 12 endoscopic response rate (defined as a 50% reduction in SES-CD). For endoscopic response, the assumed mirikizumab and placebo rates are 35% and 15%, respectively.

Treatment comparisons of the primary endpoint and other categorical efficacy variables may be conducted using a logistic regression analysis with treatment, geographic region, and prior biologic CD therapy use (yes/no) in the model. Unless otherwise specified, efficacy and health outcomes analyses may be conducted on the intent-to-treat population (ITT).
A Phase II study essentially as described above in this Example 1 was performed.

### Results: Patient Population

### Period 1

There were 191 subjects in the intent to treat (ITT) population: 64 subjects in the placebo group, 31 subjects in the mirikizumab 200 mg IV group, 32 subjects in the mirikizumab 600 mg IV group, and 64 subjects in the mirikizumab 1000 mg IV group.

Of the 191 subjects who received at least 1 dose, 176 subjects (92.1%) completed Period 1 while 15 subjects (7.9%) did not complete Period 1. Early discontinuations were balanced across the treatment groups with the exception of the mirikizumab 600 mg IV group (placebo 7.8%, 200 mg IV 6.5%, 600 mg IV 12.5%, 1000 mg IV 6.3%). The higher rate of discontinuation noted in the 600 mg IV group was reflected in the rates of discontinuation for AE. Of the 15 subjects who discontinued early, 8 (4.2%) discontinued early due to an AE: 4 subjects (6.3%) in the placebo group, 1 subject (3.2%) in the mirikizumab 200 mg IV group, and 3 subjects (9.4%) in the mirikizumab 600 mg IV group. Early discontinuations from the study due to an AE are further discussed in Section 1.9 of this appendix. Two subjects in the mirikizumab 1000 mg IV group discontinued early because of subject decision ("withdrawal by subject").

### Period 2

Of the 176 subjects who continued treatment in Period 2, 28 subjects had discontinued as of the interim analysis database lock date. Of the 28 subjects who discontinued by the end of Period 2 (Week 52), 11 discontinued due to an AE: 1 subject in the mirikizumab 1000 mg IV group, 1 subject in the mirikizumab 300 mg SC group, 3 subjects in the mirikizumab 1000 mg IV for NI group, and 6 subjects in the placebo/1000 mg mirikizumab IV group. Eight subjects discontinued early because of subject decision ("withdrawal by subject").

Of the 176 subjects who continued treatment in Period 2, 39 subjects had discontinued as of the final analysis database lock date. Of the 39 subjects who discontinued by the end of Period 2 (Week 52), 12 discontinued due to an AE: 1 subject in the mirikizumab 1000 mg IV group, 1 subject in the mirikizumab 300 mg SC group, 3 subjects in the mirikizumab 1000 mg IV for NI group, and 7 subjects in the placebo/1000 mg mirikizumab IV group.

### Results: Demographics and Disease Characteristics

### Demographic Characteristics

Demographic characteristics were balanced between the total mirikizumab group and placebo. Of the 191 randomized subjects, 98 subjects (51.3%) were female. The mean age (±standard deviation) was 38.65 years (± 12.86 years). A total of 159 subjects (83.2%) were white.

The mean baseline weight was 72.71 kg (±15.71 kg). The mean baseline body mass index (BMI) was 25.18 kg/m2 (±4.88 kg/m2).

### Disease Characteristics

Disease characteristics were balanced between the total mirikizumab treatment group and individual dosing groups and placebo. Overall, 62.8% of subjects had been previously exposed to biologic therapies, and this percentage was balanced across the treatment groups. Important baseline disease characteristics, such as disease duration, prior biologic use and disease activity (CDAI, endoscopic score and PROs) were generally balanced across the 4 treatment groups. The percentage of patients with a history of resection in the mirikizumab 200 mg group was lower than the other treatment groups.

### Concomitant and Prior Medications for CD at Baseline

The concomitant and prior CD medications at Period 1 baseline are shown in **Table 1a.**

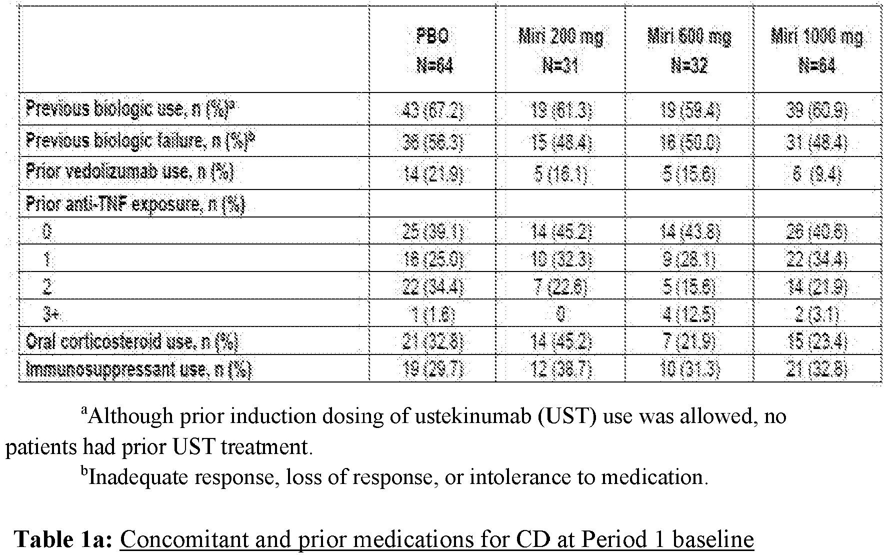

There were no meaningful differences between the total mirikizumab group or individual dosing groups and placebo with regard to the proportion of subjects receiving corticosteroids or immunosuppressants at baseline.

The concomitant and prior CD medications at Period 2 are shown in **Table 1b.**

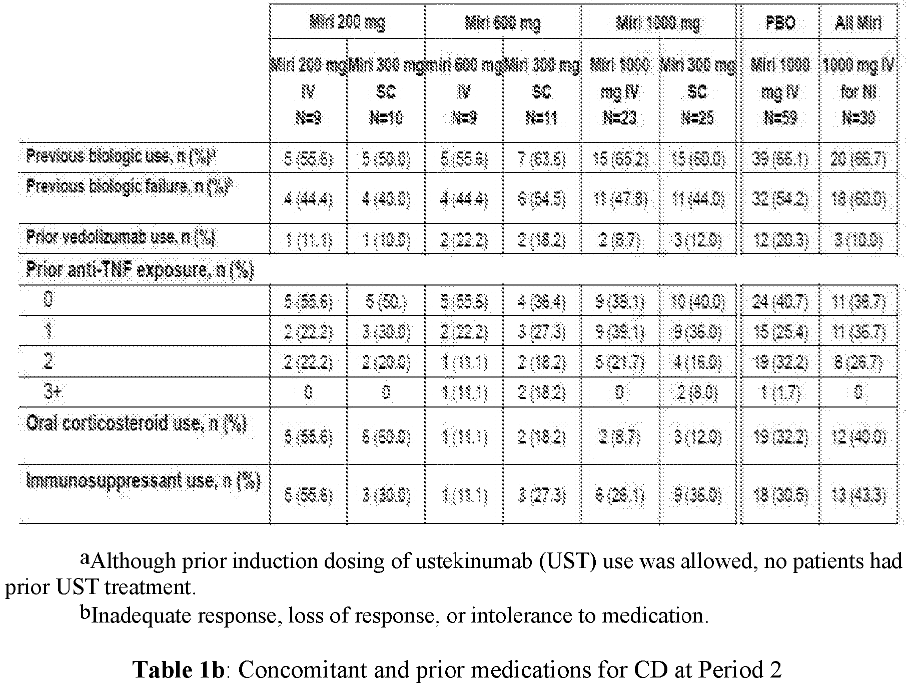

### Results: Efficacy

### Period 1

The primary endpoint was endoscopic response (defined as a 50% reduction in SES-CD) at Week 12. The data show increased (or improved) efficacy with increasing mirikizumab dose: 10.9%, 95% CI (3.3%, 18.6%) of subjects in the placebo group, 25.8%, 95% CI (10.4%, 41.2%) of subjects in the mirikizumab 200 mg IV, 37.5%, 95% CI (20.7%, 54.3%) of subjects in the mirikizumab 600 mg IV, and 43.8%, 95% CI (31.6%, 55.9%) of subjects in the mirikizumab 1000 mg IV) attaining the endpoint of endoscopic response. In a subgroup analysis of bio-experienced subjects, the proportion of subjects with endoscopic response at Week 12, with the 1000 mg dose compared to the 600 mg dose was numerically greater, with 46.2% versus 31.6% responding.

Clinical remission was assessed at Week 12 using a definition based on PRO. Clinical remission by PRO (2.5,1) was defined in this study as SF ≤ 2.5 and AP ≤ 1 and no worse than baseline. The proportion of subjects with clinical remission by PRO (2.5, 1) was significantly higher for the 600 mg and 1000 mg doses compared with placebo with the remission rate for 600 mg (28.1%, 95% CI [12.5%, 43.7%]) numerically higher than 1000 mg (21.9%, 95% CI [11.7%, 32.0%]). Using clinical remission by PRO (3,1) defined as SF ≤ 3.0 and AP ≤ 1 and no worse than baseline, the cut-off definition in Example 2, the proportion of subjects with clinical remission was significantly higher for the 600 mg and 1000 mg doses compared with placebo with the remission rate for the 600 mg dose (28.1%, 95% CI [12.5%, 43.7%]) was the same as the 1000 mg dose (28.1%, 95% CI [17.1%, 39.1%]).

A summary of efficacy measures at Week 12 are presented in **Table 2.**

**Table 2: Summary of Efficacy Measures at Week 12**

| | | | **PBO IV Q4W (N = 64)** | **Miri IV Q4W 200 mg (N = 31)** | **Miri IV Q4W 600 mg (N = 32)** | **Miri IV Q4W 1000 mg (N = 64)** |
|---|---|---|---|---|---|---|
| **Endoscopic Response^{a}** | | | | | | |
| | Nx | | 59 | 29 | 29 | 60 |
| | n (%) | | 7 (10.9) | 8 (25.8) | 12 (37.5) | 28 (43.8) |
| | 95% CI^{b} | | (3.3, 18.6) | (10.4, 41.2) | (20.7, 54.3) | (31.6, 55.9) |
| | Difference vs placebo | | | 14.9% | 26.6% | 32.8% |
| | 95% CI^{b} | | | (-2.3, 32.1) | (8.1, 45.0) | (18.5, 47.2) |
| | p-value vs placebo^{c} | | | 0.079* | 0.003* | <0.001 * |

| | **Endoscopic response among bio-naïve subjects** | | | | | |
|---|---|---|---|---|---|---|
| | | N | 21 | 12 | 13 | 25 |
| | | n (%) | 2 (9.5) | 5 (41.7) | 6 (46.2) | 10 (40.0) |
| | | 95% CI^{b} | (0.0, 22.1) | (13.8, 69.6) | (19.1, 73.3) | (20.8, 59.2) |
| | | Difference vs placebo | | 32.1% | 36.6% | 30.5% |
| | | 95% CI^{b} | | (1.6, 62.7) | (6.8, 66.5) | (7.5, 53.4) |
| | | p-value vs placebo^{c} | | 0.071* | 0.033* | 0.041* |
| | | N | 43 | 19 | 19 | 39 |
| | | n (%) | 5 (11.6) | 3 (15.8) | 6 (31.6) | 18 (46.2) |
| | | 95% CI^{b} | (2.0,21.2) | (0.0, 32.2) | (10.7, 52.5) | (30.5, 61.8) |
| | | Difference vs placebo | | 4.2% | 20.0% | 34.5% |
| | | 95% CI^{b} | | (-14.8,23.2) | (-3.0, 42.9) | (16.2, 52.9) |
| | | p-value vs placebo^{c} | | 0.692 | 0.077* | <0.001* |

| **Endoscopic remission**^{d} | | | | | | |
|---|---|---|---|---|---|---|
| | Nx | | 59 | 29 | 29 | 60 |
| | n (%) | | 1 (1.6) | 2 (6.5) | 5 (15.6) | 13 (20.3) |
| | 95% CI^{b} | | (0.0, (4.6) | (0.0, 15.1) | (3.0, 28.2) | (10.5, 30.2) |
| | Difference vs placebo | | | 4.9% | 14.1% | 18.8% |
| | 95% CI^{b} | | | (-4.3, 14.1) | (1.1, 27.0) | (8.4, 29.1) |
| | p-value vs placebo^{c} | | | 0.241 | 0.032* | 0.009* |

| **Clinical remission by PRO (2.5, 1)^{e}** | | | | | | |
|---|---|---|---|---|---|---|
| | Nx | | 58 | 26 | 29 | 56 |
| | n (%) | | 4 (6.3) | 4 (12.9) | 9 (28.1) | 14 (21.9) |
| | 95% CI^{b} | | (0.3, 12.2) | (1.1, 24.7) | (12.5, 43.7) | (11.7, 32.0) |
| | Difference vs placebo | | | 6.7% | 21.9% | 15.6% |
| | 95% CI^{b} | | | (-6.6, 19.9) | (5.2, 38.5) | (3.9, 27.4) |
| | p-value vs placebo^{c} | | | 0.346 | 0.005* | 0.025* |

| **Clinical remission by PRO (3, 1)^{f}** | | | | | | |
|---|---|---|---|---|---|---|
| | Nx | | 58 | 26 | 29 | 56 |
| | n (%) | | 6 (9.4%) | 5 (16.1) | 9 (28.1) | 18 (28.1) |
| | 95% CI^{b} | | (2.2, 16.5) | (3.2,29.1) | (12.5, 43.7) | (17.1, 39.1) |
| | Difference vs placebo | | | 6.8% | 18.8% | 18.8% |
| | | 95% CI^{b} | | (-8.0, 21.5) | (1.6, 35.9) | (5.6%, 31.9%) |
| | | p-value vs placebo^{c} | | 0.402 | 0.020* | 0.014* |

| | | | | | | |
|---|---|---|---|---|---|---|
| Abbreviations: AP = abdominal pain CI = confidence interval; ITT = intent-to-treat population; IV = intravenous; Miri = mirikizumab; n = number of subjects in the specified category; NRI = non-responder imputation; Nx = number of subjects in the analysis with non-missing data; PBO = placebo; PRO = patient-reported outcome; Q4W = every 4 weeks; SES-CD = Simple Endoscopic Score for CD; SF = stool frequency; vs = versus. ^{a} Endoscopic response defined as a decrease in SES-CD from baseline ≥50%. ^{b} Confidence intervals are calculated using Wald method. ^{c} Logistic regression analysis with geographic region and prior biologic experience as factors. ^{d} Endoscopic remission defined as total score SES-CD ≥4 with no subscore >1. ^{e} Clinical remission by PRO (2.5,1): SF ≤2.5 and AP ≥1 and no worse than baseline. ^{f} Clinical remission by PRO (3,1): SF ≤3 and AP ≤1 and no worse than baseline. *p<0.10. | | | | | | |

### Period 2

### a) Re-randomized Group

Subjects who received mirikizumab and who achieved any improvement in their SES CD score from baseline at Week 12 are randomized to either continue Period 1 treatment assignment (mirikizumab 1000 mg IV, 600 mg IV, or 200 mg IV Q4W with placebo administered SC OR placebo IV Q4W with mirikizumab 300 mg SC Q4W). This re-randomization is designed to address the question of whether there are benefits to continued IV dosing compared to SC dosing, as well as to evaluate the possible differences in efficacy between various dosing groups, representing a wide range of exposures.

At database lock for the interim analysis, 46 of 191 enrolled subjects in the re-randomized group had either completed Week 52 or discontinued early (200/200 mg IV - 5; 600/600 mg IV - 6; 1000/1000 mg IV - 12; and 300 mg SC - 23). Efficacy measures at Week 52 are summarized in **Table 3.**

Endoscopic response is observed in 50% to 66.7% of patients in this re-randomized group overall. The endoscopic response rate in patients receiving 300 mg SC was 65.2%, which is comparable to the rates observed for the other IV dosing groups. The rate of endoscopic remission is 16.7% to 33.3% (200 mg IV - 20.0%, 600 mg IV - 16.7%, 1000 mg IV - 33.3%), compared to 34.8% among patients receiving 300 mg SC. Exposures increased as expected between the 300 SC regimen versus the IV regimens and as expected with increasingly higher IV regimens.

Clinical remission by PRO, whether assessed with the SF cutoff of 2.5 or 3.0 show the same results. The percentage of patients in clinical remission by PRO is similar in the 600 mg IV, 1000 mg IV, and 300 mg SC dosing groups (25.0% to 33.3%) and higher for the 200 mg IV group (80%).

At database lock for the final analysis, a total of 87 patients were re-randomized and either completed Week 52 or discontinued early:

| | | | |
|---|---|---|---|
| i) | 200/200 mg IV | - | n = 9 patients, 2 discontinued; |
| ii) | 600/600 mg IV | - | n = 9 patients, 1 discontinued; |
| iii) | 1000/1000 mg IV | - | n = 23, 5 discontinued; and |
| iv) | 300 mg SC | - | n = 46, 5 discontinued. |

Efficacy measures at Week 52 are summarized in **Table 4.** For endoscopic improvers at Week 12, the rates of endoscopic response at Week 52 for the IV and SC groups were 58.5% (24/41) and 58.7% (27/46), respectively. Among those with endoscopic response at Week 12, 69.6% (16/23) and 66.7% (16/24) in the IV-C and SC groups, respectively, also had endoscopic response at Week 52. Other secondary and exploratory endpoints are reported for the combined randomized groups (IV+SC) **(Table 4).**

The results from this re-randomized group support comparable efficacy of SC dosing to IV dosing, and a lack of any clear change in efficacy with the increasing exposures seen with IV dosing.

### b) Non-randomized Group

Subjects who had received mirikizumab and who did not achieve an improvement in their SES CD score receive IV mirikizumab 1000 mg and SC placebo in Period 2. In addition, all subjects who received placebo in Period 1 received IV mirikizumab 1000 mg and SC placebo in Period 2. These patients are assigned to the highest IV dose to assess any effect of the greatest exposures on patients with an initial lack of endoscopic improvement and to assess the effect of shorter duration of exposure (9 months) on patients who had previously been randomized to placebo. For these reasons, the patients in these 2 groups represent separate patient populations, with different underlying baseline characteristics compared to patients re-randomized to 1000 mg IV due to the 12-week duration of either absence of endoscopic improvement or untreated disease. Therefore, in evaluating long-term exposure to 1000 mg IV, these groups are analyzed separately.

At database lock for the interim analysis, 46 of 191 enrolled subjects had either completed Week 52 or discontinued early (1000 mg IV/NI - 14, placebo/1000 mg IV - 32).

Endoscopic response was observed in 14.3% of subjects who had not improved (NI) in Period 1 and in 46.9% of patients who had received placebo in Period 1. The latter result was comparable to the rate of endoscopic response observed in the re-randomized dosing groups, while the rate observed in those with no endoscopic improvement was lower. Endoscopic remission paralleled the results for endoscopic response, with the rate of endoscopic remission rate in the patient who had not improved endoscopically and then received 1000 mg IV was 7.1%, while the endoscopic remission rate for patients receiving placebo followed by 1000 mg IV was 15.6%.

The results for clinical remission by PRO (2.5, 1) at Week 52 was 21.4% for the 1000 mg IV/NI group and 34.4% for patients who were in the placebo/1000 mg group. Results for clinical remission by PRO (3.0, 1) were similar.

At database lock for the final analysis, a total of 89 patients who were no-improvers or received placebo up to Week 12 received treatment to Week 52 or discontinued as follows:

| | | | |
|---|---|---|---|
| i) | Non-improvers 1000 mg IV | - | n = 30 patients, 7 discontinued |
| | | | a) 200 mg IV Period 1: 10/29 (34.5%) |
| | | | b) 600 mg IV Period 1: 8/29 (27.6%) |
| | | | c) 1000 mg IV Period 1: 12/60 (20.0%) |
| ii) | Placebo 1000 mg IV | - | n = 59 patients, 19 discontinued |

Endoscopic response was observed in 20% of subjects who had not improved (NI) in Period 1 and in 42.4% of patients who had received placebo in Period 1. Endoscopic remission paralleled the results for endoscopic response. The rate of endoscopic remission in the patients who had not improved endoscopically and then received 1000 mg IV was 13.3%, while the endoscopic remission rate for patients receiving placebo followed by 1000 mg IV was 18.6%.

The results for clinical remission by PRO (2.5, 1) at Week 52 was 36.7% for the 1000 mg IV/NI group and 40.7% for patients who were in the placebo/1000 mg group. Results for clinical remission by PRO (3.0, 1) were similar.

Results for the NI and PBO groups were comparable to the combined IV and SC groups except for PRO remission, CDAI remission, and endoscopic endpoints in the NI group, which were numerically lower. CDAI score decreased throughout the maintenance period.

**Table 3: Summary of Efficacy Measures at Week 52; Period 2 - Interim Analysis**

| | | **Miri 200 mg IV/200 mg IV Q4W (N =5)a** | **Miri 600 mg IV/600 mg IV Q4W (N = 6)** | **Miri 1000 mg IV/1000 mg IV (N = 12)** | **Miri IV/300 mg SC Q4W (N = 23)** | **Miri 1000 mg IV Q4W for NI (N = 14)** | **PBO/1000 mg IV Q4W (N = 32)** |
|---|---|---|---|---|---|---|---|
| **Endoscopic response^{b}** | | | | | | | |
| | Nx | 4 | 5 | 9 | 20 | 9 | 20 |
| | n (%) | 3 (60) | 4 (66.7%) | 6 (50.0) | 15 (65.2) | 2 (14.3) | 15 (46.9) |
| | 95% CI | (17.14, 100) | (28.9, 100) | (21.7, 78.3) | (45.8, 84.7) | (0, 32.6) | (29.6, 64.2) |

| **Endoscopic remission^{c}** | | | | | | | |
|---|---|---|---|---|---|---|---|
| | Nx | 4 | 5 | 9 | 20 | 9 | 20 |
| | n (%) | 1 (20.0) | 1 (16.7) | 4 (33.3) | 8 (34.8) | 1 (7.1) | 5 (15.6) |
| | 95% CI | (0, 55.1) | (0, 46.5) | (6.7, 60.0) | (15.3, 54.2) | (0, 20.6) | (3.0, 28.2) |

| **Clinical remission by PRO (2.5, 1)^{d}** | | | | | | | |
|---|---|---|---|---|---|---|---|
| | Nx | 4 | 5 | 8 | 17 | 8 | 19 |
| | n (%) | 4 (80.0) | 2 (33.3) | 3 (25.0) | 6 (26.1) | 3 (21.4) | 11 (34.4) |
| | 95% CI^{b} | (44.9, 100) | (0, 71.1) | (0.5, 49.5) | (8.1, 44.0) | (0, 42.9) | (17.9, 50.8) |

| **Clinical remission by PRO (3, 1)^{e}** | | | | | | | |
|---|---|---|---|---|---|---|---|
| | Nx | 4 | 5 | 8 | 17 | 8 | 19 |
| | n (%) | 4 (80.0) | 2 (33.3) | 3 (25.0) | 6 (26.1) | 4 (28.6) | 11 (34.4) |
| | 95% CI | (44.9, 100) | (0, 71.1) | (0.5, 49.5) | (8.1, 44.0) | (4.9,52.2) | (17.9, 50.8) |

| | | | | | | | |
|---|---|---|---|---|---|---|---|
| Abbreviations: CI = confidence interval; IV = intravenous; Miri = mirikizumab; N = number of subjects who either discontinued during the maintenance period or had data available for the Week 52 visit; n = number of subjects in the specified category; PRO = patient-reported outcome; Q4W = every 4 weeks; SC = subcutaneous; SES-CD = Simple Endoscopic Score for Crohn's Disease. Notes: Confidence intervals were calculated using Wald's method. ^{a} N includes all patients who have either completed or discontinued the study. ^{b} Endoscopic response defined as a decrease in SES-CD from baseline ≥50%. ^{c} Endoscopic remission defined as total score SES-CD ≥4 with no subscore >1. ^{d} Definitions are: Clinical remission by PRO (2.5.1): SF ≤2.5 and AP ≤1 and no worse than baseline. ^{e} Proposed endpoint definition in Phase 3. Clinical remission by PRO (3,1): SF ≤3 and AP ≤1 and no worse than baseline. | | | | | | | |

**Table 4: Summary of Efficacy Measures at Week 52; Period 2 - Final Analysis**

| | **Induction improvers re-randomized to Maintenance Dosing** | | | | | | | | **Induction NI** | **Induction PBO** |
|---|---|---|---|---|---|---|---|---|---|---|
| | **Induction - 200 mg IV** | | **Induction - 600 mg IV** | | **Induction - 1000 mg IV** | | **Induction - All IV** | | **1000 mg IV n=30** | **1000 mg IV n=59** |
| | **200 mg IV n=9** | **300 mg SC n=10** | **600 mg IV n=9** | **300 mg SC n=10** | **1000 mg IV n=23** | **300 mg SC n=25** | **All IV n=41** | **300 mg SC n=46** | | |
| SES-CD response^{a}, n (%) | 4 (44.4) | 5 (50.0) | 7 (77.8) | 8 (72.7) | 13 (56.0) | 14 (56.0) | 24 (58.5) | 27 (58.7) | 6 (20.0) | 25 (42.4) |
| SES-CD remission^{b}, n (%) | 0 (0.0) | 4 (40.0) | 2 (22.2) | 4 (36.4) | 6 (26.1) | 7 (28.0) | 8 (19.5) | 15 (32.6) | 4 (13.3) | 11 (18.6) |
| SES-CD remission adjusted^{c}, n (%) | 1 (11.1) | 4 (40.0) | 2 (22.2) | 4 (36.4) | 6 (26.1) | 7 (28.0) | 8 (19.5) | 15 (32.6) | 4 (13.3) | 11 (18.6) |
| PRO remission^{d}, n (%) | 7 (77.8) | 5 (50.0) | 5 (55.6) | 5 (45.5) | 7 (30.4) | 11 (44.0) | 19 (46.3) | 21 (45.7) | 11 (36.7) | 24 (40.7) |
| PRO (3,1) remission^{e}, n (%) | 7 (77.8) | 6 (60.0) | 5 (55.6) | 5 (45.5) | 8 (34.8) | 11 (44.0) | 20 (48.8) | 22 (47.8) | 12 (40.0) | 24 (40.7) |
| PRO response^{f}, n (%) | 7 (77.8) | 7 (70.0) | 6 (66.7) | 9 (81.8) | 15 (65.2) | 17 (68.0) | 28 (68.3) | 33 (71.7) | 18 (60.0) | 36 (61.0) |
| CDAI responses, n (%) | 7 (77.8) | 7 (70.0) | 5 (55.6) | 10 (90.9) | 10 (43.5) | 15 (60.0) | 22 (53.7) | 32 (69.6) | 14 (46.7) | 31 (52.5) |
| CDAI remission^{h}, n (%) | 7 (77.8) | 6 (60.0) | 4 (44.4) | 9 (81.8) | 5 (21.7) | 11 (44.0) | 16 (39.0) | 26 (56.5) | 7 (23.3) | 24 (40.7) |

| | | | | | | | | | | |
|---|---|---|---|---|---|---|---|---|---|---|
| ^{a}SES-CD response: 50% reduction from baseline in SES-CD Score; ^{b}SES-CD remission: SES-CD score of <4 for ileal-colonic disease or <2 for isolated ileal disease, and no subscore >1; ^{c}SES-CD remission adjusted: SES-CD score of ≤4 for ileal-colonic disease or <2 for isolated ileal disease, and no subscore >1; ^{d}PRO remission: SF ≤ 2.5 and AP ≤ 1 and no worse than baseline; ^{e}PRO (3,1) remission: SF ≤ 3 and AP ≤ 1 and no worse than baseline; ^{f}PRO response: decrease of 30% or more in AP or SF with no worsening from baseline; ^{g}CDAI response: decrease from baseline in CDAI Score of 100 points or more or a CDAI score < 150; ^{h}CDAI remission: A CDAI score of <150 points. NI = Non-improvers (no change or at least 1-point increase in SES-CD total score). | | | | | | | | | | |

### Results: Safety

Overviews of adverse events (AE)s by study periods are presented in **Table 5a** for Period 1 and **Table 5b** for Period 2.

**Table 5b: Overview of Adverse Events Safety Population, Period 2 (Weeks 13-52)**

| | | **PBO IV Q4W** | **Miri IV Q4W 200 mg** | **Miri IV Q4W 600 mg** | **Miri IV Q4W 1000 mg** | **Miri Total** |
|---|---|---|---|---|---|---|
| | | **(N = 64)** | **(N = 31)** | **(N = 32)** | **(N = 64)** | **(n = 127)** |
| **Number of Subjects^{a}** | | **n (%)** | **n (%)** | **n (%)** | **n (%)** | **n (%)** |
| | TEAEs | 45 (70.3) | 18 (58.1) | 21 (65.6) | 42 (65.6) | 81 (63.8) |
| | SAEs | 7 (10.9) | 0 (0.0) | 3 (9.4) | 2 (3.1) | 5 (3.9) |
| | Discontinuations from study due to an AE | 3 (4.7) | 1 (3.2) | 3 (9.4) | 0 (0.0) | 4 (3.1) |

| | | | | | | |
|---|---|---|---|---|---|---|
| ^{a}Subjects may be counted in more than 1 category. Deaths were also included as SAEs and discontinuations due to AEs. | | | | | | |

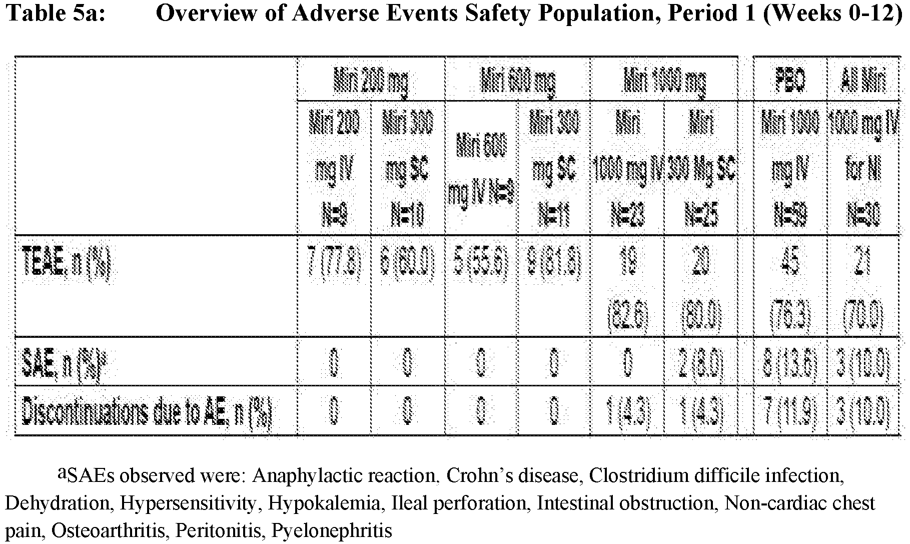

Mirikizumab was well tolerated with 4 subjects (3.1%) in Period 1 and 12 subjects (5.7%) in Period 2 discontinuing due to adverse events (AE)s. In Period 1 the incidence of serious adverse events (SAEs) was higher in the placebo group than in the mirikizumab treatment groups and no dose relationship was observed. The incidence of TEAEs was similar across placebo and mirikizumab treatment groups in Period 1 with no dose relationship observed.

The number of SAEs observed in Period 1 are higher in the cohorts that were (a) administered placebo in Period 1 and 1000 mg of mirikizumab IV in Period 2 and (b) non-improvers in Period 1 and administered 1000 mg of mirikizumab IV in Period 2. The sample size, however, does not support a conclusion that administration of 1000 mg of mirikizumab IV results in a higher incidence of SAEs, particularly when examined with SAE data from the improver cohort that was administered 1000 mg of mirikizumab IV in Period 1 and 1000 mg of mirikizumab IV in Period 2.

### Results: PK and Exposure-Response Analyses

### a) PK Analysis - summary of mirikizumab serum exposure during the induction and maintenance periods

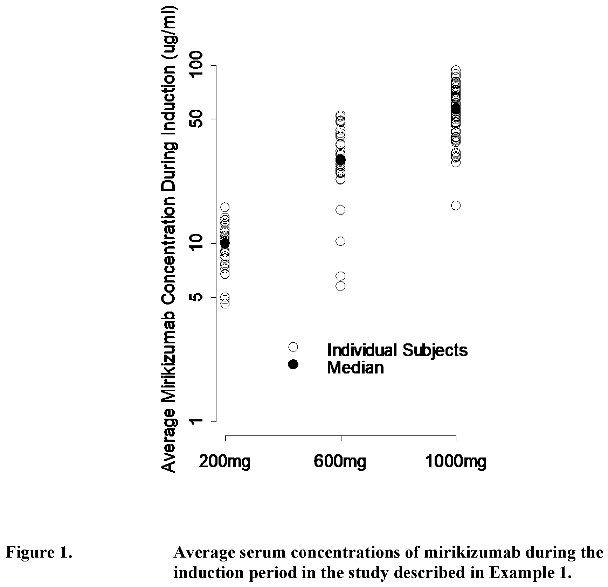

**Figure 1** shows the average concentrations of mirikizumab during Period 1 (induction period), which are calculated using the individual subject clearance estimated by the population PK analyses and the total dose each subject received during the induction period. As shown in these graphs, serum exposure of mirikizumab increased with dose, with some overlap of individual subject exposures across the doses that were evaluated. Note that some individual subjects have very low average concentrations relative to other subjects in the same treatment group. This is mainly the result of these subjects dropping out from the study and not receiving all the planned doses, which results in their low average concentrations over the entire 12-week induction period.

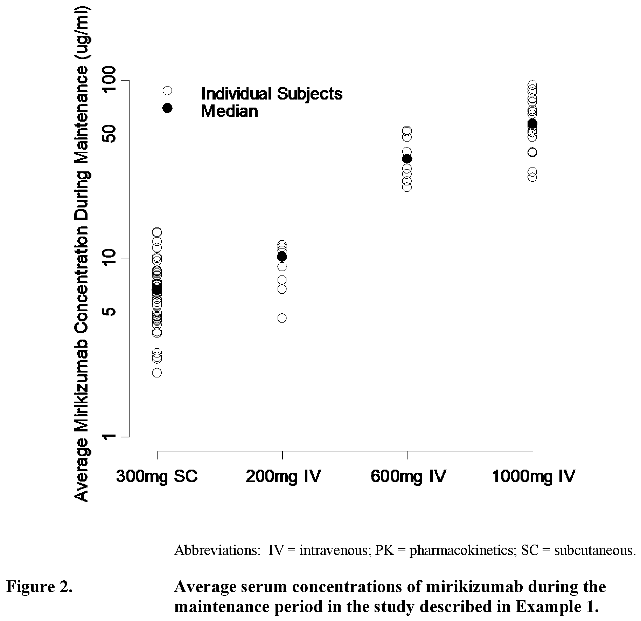

**Figure 2** shows the average concentration of mirikizumab during the maintenance period for subjects that showed improvement in endoscopic efficacy during induction and were randomized to either continue on the IV dose they received during induction or switched to 300 mg SC Q4W dosing. The 300 mg SC Q4W regimen produced the lowest average concentration of the 4 regimens that were evaluated; however, the 200 mg IV Q4W regimen produced similar exposures. The trough concentrations produced by the 300 mg SC Q4W regimen were also similar to the trough concentrations produced by the 200 mg IV Q4W regimen.

### b) Summary of Population PK Analyses

At the point of the interim analysis for this study, a total of 1814 serum mirikizumab concentration samples in 186 patients from the induction, extension, and maintenance periods were included in the PK analysis. These concentration data were analyzed using population PK methods. A 2 compartment model with first order absorption for the SC maintenance doses was found to best describe the PK of mirikizumab. The estimated typical population systemic clearance was 0.028 L/hr (4.3% standard error of the estimate), and random between patient variability in apparent clearance was 24% (% coefficient of variation). The estimated SC bioavailability was 42%. The estimated typical population values for central and peripheral volumes of distribution and inter-compartmental clearance were 3.2 L, 4.2 L, and 0.067 L/hr, respectively. A total of 24 samples (1.2%) were below the lower limit of quantitation of the mirikizumab assay (100 ng/mL). Excluding these samples was compared to standard imputation or conditional estimation methods in the PK modeling, and no impact on the estimated PK parameters was noted.

The population PK model was used to evaluate the impact of the following covariates: age, gender, BMI, body weight, ethnic origin, dose level, site of injection, prior biologic status, baseline albumin, time varying albumin, baseline C-reactive protein (CRP), baseline fecal calprotectin, baseline bilirubin, baseline SES-CD score, baseline CDAI score, baseline PRO2 score (PRO2 is a 2-item index comprised of the SF and AP items from the CDAI [weighted]) and is derived as follows: PRO2 = (7-day average of SF)*2 + (7 day average of AP score)*5, and immunogenicity (ADA+/-, TE-ADA+/-, ADA titer, neutralizing ADA+/-). Baseline SES-CD score, body weight, and time varying albumin were found to have a statistically significant impact on clearance, and body weight was found to have a statistically significant impact on central volume of distribution.

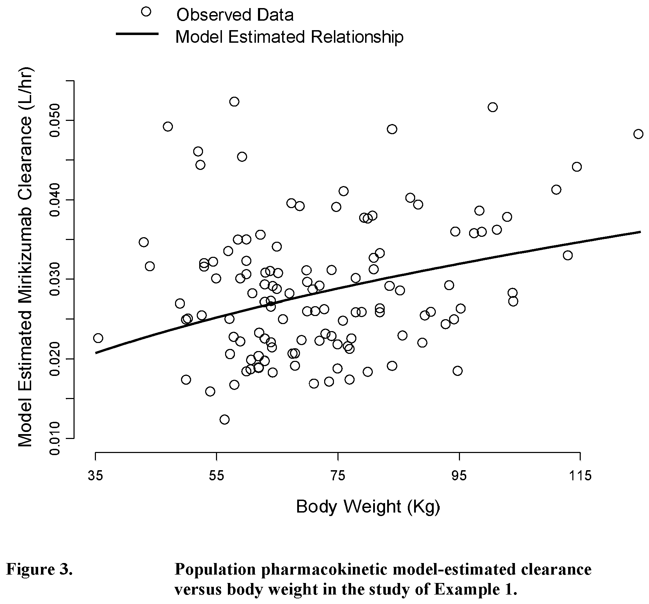

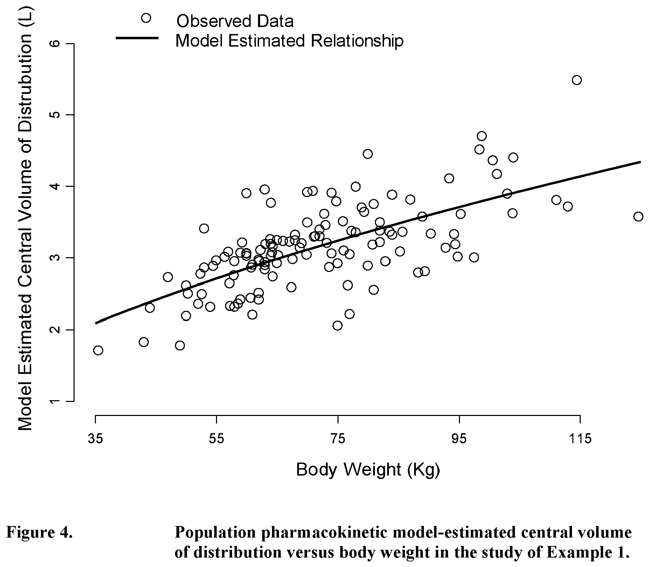

Patients with a lower body weight tended to have a lower clearance **(****Figure 3****)** and a lower central volume of distribution **(****Figure 4****).** Compared to the median body weight of 71 kg observed in this study and using the model-estimated relationships, subjects with a body weight of 40 kg would typically be expected to have a clearance that is 22% lower and a volume of distribution that is 28% lower. The magnitude of these impacts is small relative to the overall random variation in clearance and volume of distribution. Including body weight in the model reduced random variability in clearance by 14% and random variability in volume of distribution by 34%. These results indicate that body weight did not have a clinically relevant impact on the PK of mirikizumab.

Patients with a lower baseline SES-CD score tended to have a lower clearance and patients with a higher albumin concentration tended to have a lower clearance. Baseline SES-CD score also tended to increase with decreasing baseline albumin concentrations. The median baseline SES-CD score in study was 11. Patients in the study that had a baseline SES-CD score less than 11 had a median model-estimated clearance that was 23% lower than patients that had baseline SES-CD score greater than or equal to 11 (0.023 vs 0.030 L/hr). These model-estimated clearance values account for the impact of both baseline SES-CD score and the baseline albumin concentrations observed in those patients. Including baseline SES-CD score and albumin in the model reduced random variability in clearance by 25% and 17%, respectively. Based on the magnitude of the impact of baseline SES-CD score and albumin on clearance and the magnitude of the reduction in random variability in the model, these covariates did not have a clinically relevant impact on the clearance of mirikizumab.

### c) Observed relationships between mirikizumab exposure and efficacy scores at Week 52

The relationships between absolute change for SES CD, PRO2, and CDAI from Week 12 to 52 versus the PK model estimated C_{avg} during the maintenance period were assessed (data not shown). Similar to the induction period, the observed relationships show large overlap in the score changes across individual subjects in the treatment groups. There was no discernable exposure-response trend across the maintenance treatment groups.

Given the lack of an observed trend for exposure-response at Week 52, no model-based exposure-response evaluations were conducted on Week 52 data.

### d) Summary of model based Exposure-Response analyses for Week 12 clinical efficacy endpoints

Logistic regression models were used to evaluate the relationships between mirikizumab exposure in individual patients and the probability of achieving endoscopic response, endoscopic remission, or PRO remission at Week 12. Models were also used to evaluate the relationship between the change in SES-CD score at Week 12 and mirikizumab exposure. Maximum effect (Eₘₐₓ) relationships between mirikizumab exposure and these endpoints were assumed, although linear models were also tested. The exposure measures that were evaluated in the models were observed concentration at Week 12, PK model estimated concentration at Week 12, and PK model estimated C_{avg}. The following covariates were also evaluated in these models: baseline albumin, baseline CRP, baseline fecal calprotectin, prior biologic treatment status, duration of disease, baseline SES-CD, baseline CDAI, baseline SF subscore, baseline AP subscore, and body weight.

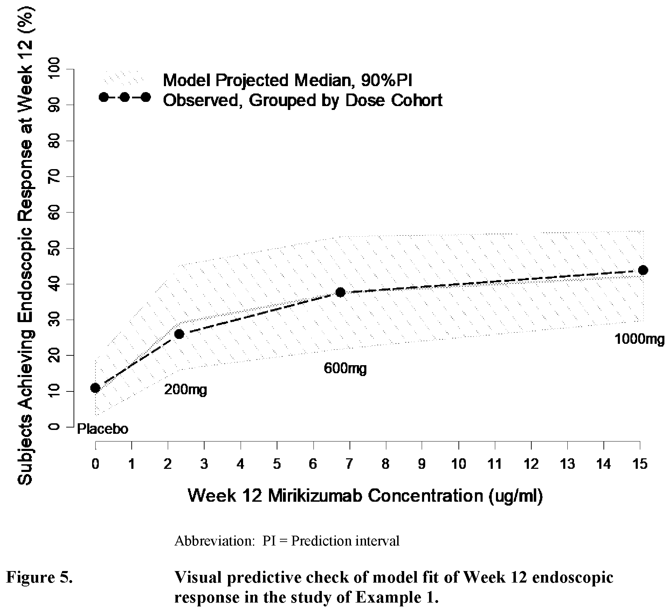

In the endoscopic response model, all the evaluated exposure measures were similar with regard to their ability to fit the observed data, with the PK model estimated Week 12 concentration providing an estimate of the half-maximal effect concentration (EC₅₀) with the lowest uncertainty. The endoscopic response model was able to detect a significant treatment effect relative to placebo (p < 0.001) and a significant exposure-response (p = 0.003). None of the evaluated covariates were found to have a significant impact in the endoscopic response model. A visual predictive check was used to validate the model **(****Figure 5****)** and it showed good agreement between the observed and model predicted endoscopic response rates across the treatment groups.

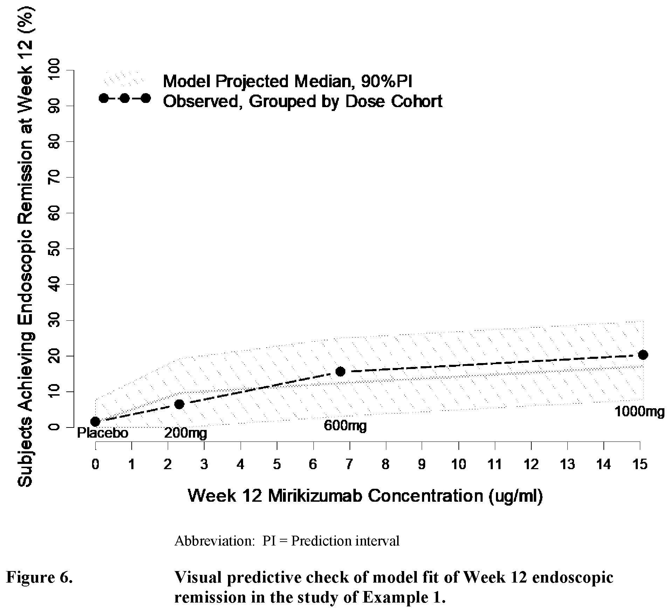

In the endoscopic remission model, the PK model estimated Week 12 concentration provided an estimate of the EC₅₀ with the lowest uncertainty. The endoscopic remission model was able to detect a significant treatment effect relative to placebo (p < 0.001) and a strong trend for exposure-response (p = 0.03). None of the evaluated covariates were found to have a significant impact in the endoscopic remission model. A visual predictive check was used to validate the model **(****Figure 6****)** and it showed good agreement between the observed and model-predicted endoscopic remission rates across the treatment groups.

The model fit of the change in the SES-CD score at Week 12 did not detect a significant relationship between mirikizumab exposure and change in the SES-CD score. The model fit of PRO remission at Week 12 also did not detect a significant relationship between mirikizumab exposure and PRO remission. Since there was no significant exposure-response relationship detected for these endpoints, model fit and simulated profiles are not shown.

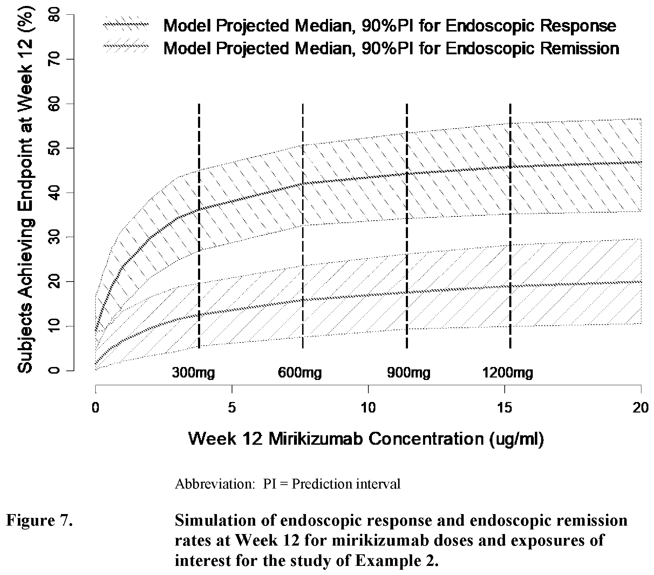

**Figure 7** shows the simulated endoscopic response and endoscopic remission rates for mirikizumab doses and exposures of interest for Phase III.

### e) Summary of PK and Exposure-Response Analyses

The PK of mirikizumab in this study was dose proportional, consistent with earlier studies and typical for a monoclonal antibody. Although serum albumin, baseline SES-CD score, and body weight were statistically significant factors that influenced mirikizumab PK, the magnitude of the impact of these factors relative to random PK variability and the observation that efficacy in individual patients was not strongly dependent on exposures within the range of interest in the study of Example 2 suggest that these patient factors will not have a clinically relevant impact on PK or efficacy in the study of Example 2.

Examination of the relationship between observed individual patient mirikizumab exposures and Week 12 SES-CD, PRO, and CDAI scores in this dtudy and model based analysis of the Week 12 endoscopic response suggest that near-maximal efficacy was achieved between the 600- and 1000 mg doses. Model-based analyses of the relationships between mirikizumab exposure and PRO remission suggest that the probability of a subject achieving PRO remission was not strongly dependent on mirikizumab exposure within the range of exposures that were evaluated in this study. In the maintenance period, the evaluated dose regimens produced a wide range of mirikizumab exposures. There was no discernable relationship between mirikizumab exposure and Week 52 efficacy across the maintenance treatment groups.

### EXAMPLE 2: CLINICAL STUDY

### Overview

A Phase III, multi-centre, randomized, double-blind, double-dummy, parallel group, active- and placebo-controlled, treat-through design study of mirikizumab may be conducted in patients with moderate-to-severe CD. More specifically, three intervention groups in the first period and four intervention groups in the second period may be studied in participants with moderate-to-severe CD:
- Mirikizumab 900 mg intravenously every 4 weeks for 3 doses, then 300 mg subcutaneously every 4 weeks;
- Ustekinumab ~ 6 mg/kg intravenously for one dose, then 90 mg subcutaneously every 8 weeks;
- Placebo
   - When Period 1 concludes (Week 12), responders continue receiving placebo; and
   - Non-responders (NR) at Week 12 receive mirikizumab as described above.
The total duration of the combined treatment periods is up to 52 weeks. The maximum total duration of study participation for each participant, including screening and the post-treatment follow-up period, is 72 weeks.

Participants in either active group receive placebo to match the other active group using a double-dummy design. Participants in the placebo group receive both double-dummy placebo administrations.

### Objectives

The primary objective is to evaluate whether treatment with mirikizumab is superior to placebo in the treatment of moderate to severe CD as assessed by endoscopic response at Week 52 and clinical remission by PRO at Week 52. Secondary objectives include the following:
- To evaluate the efficacy of treatment with mirikizumab compared to placebo in endoscopic response at Week 12;
- To evaluate the efficacy of treatment with mirikizumab compared to placebo in clinical remission by PRO at Week 12;
- To evaluate the efficacy of treatment with mirikizumab compared to placebo in endoscopic remission at Week 52;
- To evaluate the efficacy of treatment with mirikizumab compared to placebo in corticosteroid-free clinical remission by PRO or endoscopic remission at Week 52;
- To evaluate the efficacy of treatment with mirikizumab compared to placebo in the stability of clinical remission by PRO through Week 52;
- To evaluate the efficacy of treatment with mirikizumab compared to placebo in the durability of endoscopic response at Week 52;
- To evaluate the efficacy of treatment with mirikizumab compared to placebo in endoscopic remission at Week 12;
- To evaluate whether mirikizumab is superior to ustekinumab in achieving endoscopic response at Week 52;
- To evaluate whether mirikizumab is superior to ustekinumab in achieving
- endoscopic remission at Week 52; and
- To evaluate whether mirikizumab is non-inferior to ustekinumab in clinical remission by CDAI at Week 52.

Endpoints may be defined using the SES-CD score. Endoscopies may be centrally read. Rates of endoscopic healing may be determined at Weeks 12 and 52. Endpoint definitions are as follows:
- Endoscopic response: having a 50% reduction from baseline in SES CD Score.
- Endoscopic remission: having an SES-CD score of <4 for ileal colonic disease or <2 for isolated ileal disease, and no subscore >1.
- Clinical remission by PRO: having an average daily AP score ≤ 1 (and not worse that baseline) and an average daily SF ≤ 3.0 (absolute number of liquid or very soft stools defined using the Bristol Stool Scale Category 6 or 7, that is, liquid or watery stools) (and not worse than baseline).

### Methods

A Phase III, multi-centre, randomized, double-blind, double-dummy, parallel group, placebo and active controlled, treat-through study to evaluate the safety and efficacy of mirikizumab compared to placebo and ustekinumab may be conducted. The study population should include participants with moderately to severely active CD who have an inadequate response to, loss of response to, or intolerance to conventional or biologic therapy for CD.

The study may be a parallel, double-blinded treatment study with three groups in Period 1 and four groups in Period 2.

Participants may be randomized in a 6:3:2 ratio to receive, respectively:
- Mirikizumab 900 mg intravenously every 4 weeks for 3 doses, then 300 mg subcutaneously every 4 weeks;
- Ustekinumab ~ 6 mg/kg intravenously for one dose, then 90 mg subcutaneously every 8 weeks
- Placebo
   - When Period 1 concludes (Week 12), responders continue receiving placebo; and
   - Non-responders (NR) to placebo at Week 12 will receive mirikizumab as described above.

To maintain blinding, participants receive placebo in a double-dummy manner. The maximum total duration of study participation for each participant is 72 weeks, across the following study periods:
i) Screening: up to 4 weeks;
ii) Intervention Period 1: 12 weeks;
iii) Intervention Period 2: 40 weeks; and
iv) Post-Treatment Follow Up: 12 to 16 weeks

### i) Screening (approximately 4 weeks)

Participants with CD may be eligible for enrolment only if they meet all of the following criteria during screening, unless otherwise specified below:
a) Patient Characteristics
   - are male or female patients ≥18 and ≤80 years of age at the time of initial screening
b) Disease Characteristics
   - have had a diagnosis of CD or fistulizing CD established at least 3 months prior to enrollment confirmed by clinical, endoscopic, and histological criteria. Note: A histopathology report supporting the diagnosis of CD must be available in the source documents prior to randomization, in order to satisfy this inclusion criterion. If a histopathology report supporting the diagnosis of CD is not available in the source documents prior to randomization, the investigator can obtain additional biopsies for this purpose at the screening endoscopy (sent to the local histopathology laboratory).
   - have moderately to severely active CD as defined by unweighted daily average SF ≥4 (loose and watery stools defined as Bristol Stool Scale Category 6 or 7) AND/OR unweighted daily average AP ≥2 at baseline (Visit 2).
   - have a centrally read SES-CD score ≥7 for patients with ileal-colonic or ≥4 for patients with isolated ileal disease within 14 days before the first dose of study treatment.
   - Participants with a family history of colorectal cancer, personal history of increased colorectal cancer risk, age >50 years, or other known risk factor must be up-to date on colorectal cancer surveillance per local guidelines. If not, this documentation of negative colorectal cancer surveillance may be performed according to local guidelines during screening.
c) Prior Medication Failure Criteria
   - Participants must have an inadequate response to, loss of response to, or intolerance to at least one of the medications described in Inclusion Criterion [A] OR [B]. For the relevant medication specified in these criteria, documentation of dose, frequency, route of administration, and duration of the qualifying failure is required.
      [A] Conventional-failed patients: Patients who have an inadequate response to, loss of response to, or are intolerant to at least one of the following medications:
         - corticosteroids
            - corticosteroid-refractory disease, defined as signs and/or symptoms of active CD despite oral prednisone (or equivalent) at doses of at least 30 mg/day for a minimum of 4 weeks.
            - corticosteroid-dependent disease, defined as:
               a. an inability to reduce corticosteroids below the equivalent of prednisone 10 mg/day or budesonide below 3 mg/day within 3 months of starting corticosteroids without a return of signs and/or symptoms of active CD; or
               b. a relapse within 3 months of completing a course of corticosteroids.
            - history of intolerance of corticosteroids (which includes evidence of a side-effect sufficiently serious as to precluding continued treatment with corticosteroids including, but not limited to, Cushing's syndrome, osteopenia/osteoporosis, hyperglycemia, or neuropsychiatric side-effects, including insomnia, associated with corticosteroid treatment).
         - immunomodulators:
            - signs and/or symptoms of persistently active disease despite at least 3 months' treatment with one of the following:
               - oral AZA (≥1.5 mg/kg/day) or 6-MP (≥0.75 mg/kg/day) or methotrexate 25 mg (intramuscular [IM] or SC weekly); or
               - oral AZA or 6-MP within a therapeutic range as judged by thioguanine metabolite testing; or
               - a combination of a thiopurine and allopurinol within a therapeutic range as judged by thioguanine metabolite testing.
            - history of intolerance to at least one immunomodulator (including but not limited to nausea/vomiting, abdominal pain, pancreatitis, liver function test AND have neither failed nor demonstrated an intolerance to a biologic medication (anti-TNF antibody or anti-integrin antibody) that is approved for the treatment of CD. Discontinuation despite clinical benefit does not qualify as having failed or being intolerant to CD conventional therapy.
      [B] Biologic-failed patients: Participants who have an inadequate response to, loss of response to, or are intolerant to an approved biologic therapy for CD (such as anti-TNF antibodies or anti-integrin antibodies). Investigators must be able to document an adequate history of induction and/or maintenance dose use. Participants should fulfill 1 of the following criteria:
         - Inadequate response: Signs and symptoms of persistently active disease despite induction treatment at the approved induction dosing, that was indicated in the product label at the time of use, OR
         - Loss of response: Recurrence of signs and symptoms of active disease following prior clinical benefit during treatment with approved maintenance dosing. OR
         - Intolerance: History of intolerance to infliximab, adalimumab, certolizumab pegol, vedolizumab, natalizumab, or other approved biologics (including but not limited to infusion-related event, demyelination, congestive heart failure, or any other drug-related AE that led to a reduction in dose or discontinuation of the medication). Discontinuation despite clinical benefit does not qualify as having failed or being intolerant to CD biologic therapy.

Participants previously exposed to approved biologic therapy who do not meet Inclusion Criterion [B] must still meet Inclusion Criterion [A] in order to be eligible to participate in the study.

Participants previously exposed to investigational therapies for the treatment of CD must still meet inclusion criteria [A] OR [B].

Participants who meet both inclusion criteria [A] and [B] are considered to be "biologic-failed" for the purpose of this study.

### ii) Period 1 (Weeks 0-12)

Participants who meet all criteria for enrollment may be randomized to double-blind treatment. To achieve between-group comparability, participants may be stratified to treatment groups based upon these factors: a) biologic-failed status (yes/no); b) baseline corticosteroid use (yes/no); c) baseline SES-CD total score (<12, ≥12); d) region (North America/Europe/Other); and e) either baseline SF ≥ 7 and/or baseline AP ≥ 2.5 (yes/no). This stratification is controlled by interactive web-response system (IWRS). There are three intervention groups in Period 1:

| | |
|---|---|
| Mirikizumab Dose Arm 1 | 900 mg IV Mirikizumab Q4W |
| Ustekinumab Dose Arm 2 | ~6 mg/kg IV for one dose, then 90 mg SC Q8W |
| Placebo | Placebo administered IV Q4W |

### ii) Period 2 (Weeks 12-52)

There may be four intervention groups in Period 2:

| | |
|---|---|
| Mirikizumab Dose Arm 1 | 300 mg SC mirikizumab Q4W |
| Ustekinumab Dose Arm 2 | 90 mg SC ustekinumab Q8W |
| Placebo Non-responders at Week 12 | 900 mg IV Mirikizumab Q4W followed by 300 mg SC mirikizumab Q4W |
| Placebo Responders at Week 12 | Placebo administered SC Q4W (no rescue therapy after Week 12) |

### iii) Follow-up

Participants who complete this study may be given the option to enrol in an extension study if they are eligible. Participants who do not meet enrollment criteria for the extension study or who do not choose to participate in the extension study return for two post-treatment follow-up visits. The first such follow-up visit may be 4 weeks after the last dose. The second such follow-up visit may be from 12 to 16 weeks after the last dose.

## Claims

1. Mirikizumab for use in the treatment of CD, said treatment comprising:
a) administering three induction doses of mirikizumab to the patient by intravenous injection, wherein each induction dose comprises about 900 mg of mirikizumab and wherein the three induction doses of mirikizumab are administered at about 4 week intervals; and
b) administering maintenance dose(s) of mirikizumab to the patient by subcutaneous injection at about 4 week or about 8 week intervals, wherein the first maintenance dose is administered about 4 weeks or about 8 weeks after the last induction dose is administered and wherein each maintenance dose comprises 200 mg or 300 mg of mirikizumab,
wherein the CD is moderate to severe CD.

2. Mirikizumab for use in the treatment of CD according to claim 1, wherein the patient is conventional-failed.

3. Mirikizumab for use in the treatment of CD according to claim 1, wherein the patient is biologic-experienced.

4. Mirikizumab for use in the treatment of CD according to claim 1, wherein the patient is biologic-failed.

5. Mirikizumab for use in the treatment of CD according to any one of claims 1-4, wherein three induction doses of mirikizumab are administered at about 4 week intervals and the first maintenance dose is administered about 4 weeks after the last induction dose is administered.

## Patentansprüche

1. Mirikizumab zur Verwendung bei der Behandlung von Morbus Crohn (MC), die Behandlung umfassend:
a) Verabreichen von drei Induktionsdosen Mirikizumab an den Patienten durch intravenöse Injektion, wobei jede Induktionsdosis etwa 900 mg Mirikizumab umfasst und wobei die drei Induktionsdosen Mirikizumab in Abständen von etwa 4 Wochen verabreicht werden; und
b) Verabreichen einer oder mehrerer Erhaltungsdosen Mirikizumab an den Patienten durch subkutane Injektion in Abständen von etwa 4 Wochen oder etwa 8 Wochen, wobei die erste Erhaltungsdosis etwa 4 Wochen oder etwa 8 Wochen nach Verabreichung der letzten Induktionsdosis verabreicht wird und wobei jede Erhaltungsdosis 200 mg oder 300 mg Mirikizumab umfasst,
wobei der MC mittelschwerer oder schwerer MC ist.

2. Mirikizumab zur Verwendung bei der Behandlung von MC nach Anspruch 1, wobei bei dem Patienten eine konventionelle Therapie versagt hat.

3. Mirikizumab zur Verwendung bei der Behandlung von MC nach Anspruch 1, wobei der Patient mit einem Biologikum Erfahrung hat.

4. Mirikizumab zur Verwendung bei der Behandlung von MC nach Anspruch 1, wobei der Patient bereits unerfolgreich mit einem Biologikum behandelt wurde.

5. Mirikizumab zur Verwendung bei der Behandlung von MC nach einem der Ansprüche 1 bis 4, wobei drei Induktionsdosen Mirikizumab in Abständen von etwa 4 Wochen verabreicht werden und die erste Erhaltungsdosis etwa 4 Wochen nach Verabreichung der letzten Induktionsdosis verabreicht wird.

## Revendications

1. Mirikizumab destiné à être utilisé dans le traitement de la maladie de Crohn (MC), ledit traitement comprenant :
a) l'administration de trois doses d'induction de mirikizumab au patient par injection intraveineuse, où chaque dose d'induction comprend environ 900 mg de mirikizumab et où les trois doses d'induction de mirikizumab sont administrées à environ 4 semaines d'intervalle ; et
b) l'administration d'une ou plusieurs doses d'entretien de mirikizumab au patient par injection sous-cutanée à environ 4 semaines ou environ 8 semaines d'intervalle, où la première dose d'entretien est administrée environ 4 semaines ou environ 8 semaines après l'administration de la dernière dose d'induction et où chaque dose d'entretien comprend 200 mg ou 300 mg de mirikizumab,
où la MC est une MC modérée à sévère.

2. Mirikizumab destiné à être utilisé dans le traitement de la MC selon la revendication 1, où le patient est en échec de traitement conventionnel.

3. Mirikizumab destiné à être utilisé dans le traitement de la MC selon la revendication 1, où le patient a reçu un traitement biologique.

4. Mirikizumab destiné à être utilisé dans le traitement de la MC selon la revendication 1, où le patient est en échec de traitement biologique.

5. Mirikizumab destiné à être utilisé dans le traitement de la MC selon l'une quelconque des revendications 1 à 4, où trois doses d'induction de mirikizumab sont administrées à environ 4 semaines d'intervalle et la première dose d'entretien est administrée environ 4 semaines après l'administration de la dernière dose d'induction.
